# FASCICULE DE BREVET EUROPEEN

(11) **EP 2 039 339 B1**
(45) Date de publication et mention de la délivrance du brevet: **10.05.2017**
(21) Numéro de dépôt: 08164756.2
(22) Date de dépôt: 22.09.2008
(51) Int. Cl.: A61K 8/06, A61K 8/42, A61K 8/81, A61Q 1/00, A61Q 17/04, A61K 8/86, A61Q 19/00

(54) **Emulsion cosmétique de type huile-dans-eau**
Kosmetische Emulsion vom Typ Öl-in-Wasser-Emulsion
Oil-in-water type cosmetic emulsion

(30) Priorité: 24.09.2007 FR 0757800
(43) Date de publication de la demande: 25.03.2009
(73) Titulaire: L'Oréal, 75008 Paris (FR)
(72) Inventeur: LORANT, Raluca, 94320 THIAIS (FR)
(74) Mandataire: Nony

(56) Documents cités:
- EP-A- 1 331 000
- EP-A- 1 502 582
- EP-A2- 2 011 481
- WO-A-03/024412
- WO-A-2004/105704
- WO-A-2007/054824
- FR-A- 2 863 889

## Description

La présente invention se rapporte au domaine des compositions cosmétiques de type émulsion et plus particulièrement au domaine des compositions comprenant une phase grasse liquide texturée.

Les compositions cosmétiques comprenant une phase grasse liquide sont couramment utilisées de nos jours, notamment pour le nettoyage, le soin, le maquillage et/ou le traitement de la peau, du cheveu et du cuir chevelu. L'utilisation quotidienne de ces compositions fait que leurs utilisateurs sont toujours plus exigeants et plus sensibles à la texture et plus généralement aux propriétés organoleptiques de ces compositions.

Ainsi, il a été noté que des compositions cosmétiques qui comprennent plus de 10 % en poids de phase grasse contenant plus de 40 % en poids d'huile non volatile, s'avèrent avantageuses pour combler le manque de lipides cutanés des peaux déshydratées, mais en revanche peuvent poser des problèmes en terme de propriétés sensorielles, dans la mesure où elles sont susceptibles de générer un effet gras, accompagné parfois d'effet collant et/ou d'aspect brillant, peu apprécié par l'utilisateur.

Outre cet effet indésirable sur le plan de la perception sensorielle, ces compositions de type émulsion peuvent manifester un défaut de stabilité lorsqu'on les associe à certains matériaux. Ainsi, il a été constaté que la présence de quantités importantes de filtres UV notamment organiques dans des émulsions de ce type, peut affecter la stabilité de celles-ci au cours du temps, voire initier leur démixion. La composition devient alors inhomogène et n'est plus utilisable.

Il demeure donc à ce jour un besoin de compositions cosmétiques de type émulsion à teneur significative en phase grasse qui soient reproductibles à volonté en terme de viscosité, dénuées de tout caractère gras, collant et brillant, et dotées d'une stabilité améliorée notamment lorsqu'elles contiennent des matériaux de type filtre UV organique.

La présente invention a précisément pour objet principal de répondre à ces besoins.

A cet égard, les inventeurs ont, de manière inattendue, constaté qu'il était possible d'obtenir de telles émulsions sous réserve d'y mettre en oeuvre à titre d'émulsionnant un tensioactif géminé particulier en association avec au moins un polymère semi-cristallin spécifique.

Le document EP 1 502 582 décrit des compositions comprenant au moins un agent tensioactif géminé et au moins un polymère associatif, lesdites compositions étant fluides, émulsifiées et contenant un système photoprotecteur capable de filtrer les rayons UV.

Des documents WO 2007/054824, WO 03/024412 et WO 2004/105704 sont par ailleurs connues des compositions pour application topique huile-dans-eau comprenant au moins un tensioactif géminé.

Les documents FR 2 863 889 et EP 1 331 000 décrivent par ailleurs des compositions mettant an oeuvre des polymères semi-cristallins.

Toutefois, aucun de ces documents ne décrit ni ne suggère la mise en oeuvre de l'association conforme à l'invention dans l'architecture galénique plus particulièrement visée, à savoir comprenant plus de 10% au poids de phase grasse contenant plus de 40% en poids d'huile non volatile.

De façon plus précise, l'invention a pour objet une composition cosmétique ou dermatologique de type émulsion huile-dans-eau comprenant, dans un milieu physiologiquement acceptable, au moins un tensioactif géminé de formule (I) : 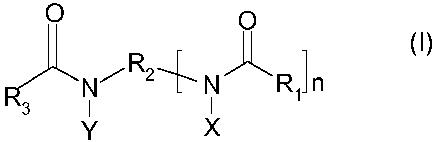 dans laquelle :
- R₁ et R₃ désignent, indépendamment l'un de l'autre, un radical alkyle ayant de 1 à 25 atomes de carbone ;
- R₂ désigne un espaceur constitué d'une chaîne alkylène linéaire ou ramifiée ayant de 1 à 12 atomes de carbone ;
- X et Y désignent, indépendamment l'un de l'autre, un groupement -(C₂H₄O)ₐ-(C₃H₆O)_{b}Z où
   - Z désigne un atome d'hydrogène ou un radical -CH₂-COOM, -SO₃M, ç_-P(O)(OM)₂, -C₂H₄-SO₃M, -C₃H₆-SO₃M ou-CH₂(CHOH)₄CH₂OH, où M représente H ou un ion alcalin ou alcalino-terreux ou ammonium ou alcanolammonium,
   - a va de 0 à 15,
   - b va de 0 à 10, et
   - la somme de a + b va de 1 à 25 ; et
- n va de 1 à 10,
en association avec une quantité efficace d'au moins un polymère semi-cristallin solide à température ambiante et ayant une température de fusion inférieure à 70°C, comportant a) un squelette polymérique et b) au moins une chaîne organique latérale cristallisable et/ou une séquence organique cristallisable faisant partie du squelette dudit polymère, ledit polymère ayant une masse moléculaire moyenne en nombre supérieure ou égale à 2 000, ledit polymère semi-cristallin étant choisi parmi les homopolymères et copolymères résultant de la polymérisation d'au moins un monomère à chaîne cristallisable choisi parmi les (méth)acrylates d'alkyle saturés en C₁₄-C₂₄ ;
ladite émulsion possédant un taux de phase grasse supérieur à 10 % en poids par rapport au poids total de ladite composition et comprenant plus de 40 % en poids d'huile(s) non volatile(s) par rapport au poids total de ladite phase grasse ;
ladite composition étant différentes des compositions A et B suivantes :

| **Ingrédients** | **Composition A** (% en poids) | **Composition B** (% en poids) |
|---|---|---|
| EDTA disodique | 0,1 | 0,1 |
| Conservateur | 1,25 | 1,25 |
| Glycérine | 4 | 4 |
| Propyleneglycol | 4 | 4 |
| Eau | 61,65 | 61,65 |
| Dispersion de particules à 86% en poids Styrene/Acrylates Copolymer (Sunspheres Powder de ROHM & HAAS) | 2 | - |
| Poly(C₁₀-C₃₀)alkylacrylates (Intelimer 13-1 de LANDEC) | 1 | 1 |
| Mélange de séarate de glycéryle, alcool béhénique, sodium sulfate de dicocoylethylendiamine PEG-15, stéarate citrate de glycéryle | 2 | 2 |
| Isononyl isononaoate | 4 | 4 |
| Alcool cétylique | 0,5 | 0,5 |
| Butylmethoxy Dibenzoylmethane | 3 | 3 |
| Ethylhexyl Salicylate | 5 | 5 |
| Octocrylene | 7 | 7 |
| Parfum | 0,4 | 0,4 |
| Tocophérol | 0,2 | 0,2 |
| Acrylates Copolymer | 0,7 | 0,7 |
| Triéthanolamine | 0,2 | 0,2 |

L'association selon l'invention permet d'obtenir des émulsions très homogènes, de stabilité accrue et donc compatibles avec la formulation de matériaux connus pour avoir naturellement un effet déstabilisant vis-à-vis des émulsions conventionnelles connues, et en particulier les filtres UV organiques.

En outre, les émulsions stabilisées par l'association conforme à l'invention sont dotées d'une texture très agréable au toucher. Elles sont douces et procurent une sensation de légèreté lors de leur application sur la peau.

Par milieu physiologiquement acceptable, on comprend un milieu compatible avec les matières kératiniques d'êtres humains comme la peau, les muqueuses, les ongles, le cuir chevelu et/ou les cheveux.

Ainsi, le milieu physiologiquement acceptable est notamment un milieu cosmétiquement ou dermatologiquement acceptable, c'est-à-dire sans odeur, couleur ou aspect désagréable, et qui ne génère pas de picotement, tiraillement ou rougeur inacceptable pour l'utilisateur.

La composition utilisée selon l'invention peut être destinée à une application cosmétique ou pharmaceutique, particulièrement dermatologique. De préférence la composition selon l'invention est destinée à une application cosmétique. Elle peut être utilisée pour le soin ou le maquillage des matières kératiniques.

Une composition selon l'invention est en particulier adaptée à une application topique sur une matière kératinique et en particulier la peau.

Ainsi, elle peut être utilisée comme produit de soin de la peau, par exemple comme crème de protection, de traitement ou de soin pour le visage, pour les mains ou pour le corps, comme lait corporel de protection ou de soin de la peau, du cuir chevelu ou des muqueuses, ou comme produit d'hygiène, par exemple comme produit de démaquillage de la peau ou des muqueuses, ou encore comme produit capillaire ou plus particulièrement comme produit solaire.

Les compositions selon l'invention peuvent constituer aussi des produits pour le maquillage de la peau et/ou des cheveux, par exemple en y incorporant des pigments pour constituer notamment des fonds de teint.

Un autre objet de l'invention est un procédé de traitement notamment cosmétique d'une matière kératinique, telle que la peau, du cuir chevelu, des cheveux, des cils, des sourcils, des ongles ou des muqueuses, caractérisé par le fait qu'on applique sur la matière kératinique, une composition telle que définie ci-dessus.

D'autres objets, caractéristiques, aspects et avantages de la présente invention apparaîtront encore plus clairement à la lecture de la description et des exemples qui suivent.

### Tensioactif géminé

Le tensioactif géminé de formule (I) est de préférence tel que chacun des groupements R₁-CO- et R₃-CO- comprenne de 8 à 20 atomes de carbone, et désigne de préférence un reste d'acides gras de coco (comprenant majoritairement de l'acide laurique et de l'acide myristique).

En outre, ce tensioactif est de préférence tel que, pour chacun des radicaux X et Y, la somme de a et b ait une valeur moyenne allant de 10 à 20 et soit de préférence égale à 15. Un groupement préféré pour Z est le groupement -SO₃M où M est de préférence un ion alcalin tel qu'un ion sodium.

L'espaceur R₂ est avantageusement constitué d'une chaîne alkylène linéaire en C₁-C₃, et de préférence d'une chaîne éthylène (CH₂CH₂).

Enfin, n est avantageusement égal à 1.

Un tensioactif de ce type est en particulier celui identifié par le nom INCI : Sodium dicocoylethylenediamine PEG-15 sulfate, ayant la structure suivante : 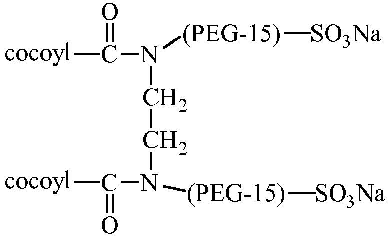 étant entendu que PEG représente le groupe CH₂CH₂O, et cocoyl représente le reste d'acides gras de coco.

Ce tensioactif possède une structure moléculaire très proche de celle de la céramide-3.

De préférence, le tensioactif géminé selon l'invention est utilisé en mélange avec d'autres tensioactifs, et notamment en mélange avec (a) un ester d'acide gras en C₆-C₂₂ (de préférence en C₁₄-C₂₀ tel qu'un stéarate) et de glycéryle, (b) un diester d'acide gras en C₆-C₂₂ (de préférence en C₁₄-C₂₀ tel qu'un stéarate) et d'acide citrique et de glycérol (notamment un diester d'acide gras en C₆-C₂₂ et du monocitrate de glycéryle), et (c) un alcool gras en C₁₀-C₃₀ (de préférence l'alcool béhénylique).

Avantageusement, la composition selon l'invention comprend un mélange de Sodium Dicocoyléthylènediamine PEG-15 Sulfate, de stéarate de glycéryle, de stéarate de monocitrate de glycéryle, d'alcool béhénylique.

Plus préférentiellement, le tensioactif géminé selon l'invention représente de 10 à 20 % en poids, et avantageusement 15 % en poids ; l'ester d'acide gras en C₆-C₂₂ et de glycéryle représente de 30 à 40 % en poids, avantageusement 35 % en poids ; le diester d'acide gras en C₆-C₂₂ et d'acide citrique et de glycérol représente de 10 à 20 % en poids, avantageusement 15 % en poids ; et l'alcool gras en C₁₀-C₃₀ représente de 30 à 40 % en poids, avantageusement 35 % en poids, par rapport au poids total du mélange de tensioactifs contenant le tensioactif géminé.

Avantageusement, la composition selon l'invention comprend un mélange de 10 à 20 % en poids de Sodium Dicocoyléthylènediamine PEG-15 Sulfate, de 30 à 40 % (en particulier 35 %) en poids de stéarate de glycéryle, de 10 à 20 % (en particulier 15 %) en poids de stéarate de monocitrate de glycéryle, de 30 à 40 % (en particulier 35 %) en poids d'alcool béhénylique, par rapport au poids total du mélange de tensioactifs contenant le tensioactif géminé.

En variante, le tensioactif géminé selon l'invention peut être utilisé en mélange avec un tensioactif anionique tel qu'un ester d'acide laurique, le lauroyl lactate de sodium. Dans ce cas, le tensioactif géminé représente de préférence de 30 à 50 % en poids, et le tensioactif anionique représente de 50 à 70 % en poids, par rapport au poids total du mélange.

On peut utiliser par exemple le tensioactif géminé en mélange avec d'autres tensioactifs sous forme des produits commercialisés par la société SASOL sous les dénominations CERALUTION^{®}, et notamment les produits suivants :
- Ceralution^{®} H : Behenyl Alcohol, Glyceryl Stearate, Glyceryl Stearate Citrate et Sodium Dicocoylethylenediamine PEG-15 Sulfate,
- Ceralution^{®} F : Sodium Lauroyl Lactylate et Sodium Dicocoylethylènediamine PEG-15 Sulfate.
- Ceralution^{®} C : Aqua, Capric/Caprylic triglyceride, Glycerin, Ceteareth-25, Sodium Dicocoylethylenediamine PEG-15 Sulfate, Sodium Lauroyl Lactylate, Behenyl Alcohol, Glyceryl Stearate, Glyceryl Stearate Citrate, Gum Arabic, Xanthan Gum, Phenoxyethanol, Methylparaben, Ethylparaben, Butylparaben, Isobutylparaben (dénominations INCI).

Ce tensioactif géminé représente de 3 à 50 % du poids de ces mélanges.

Le tensioactif géminé de formule (I) peut être présent dans une composition selon l'invention en une teneur allant de 0,01 à 5 % en poids, par rapport au poids total de la composition, de préférence allant de 0,1 à 3 % en poids et mieux allant de 0,2 à 1,5 % en poids.

### Polymère semi-cristallin

Le polymère semi-cristallin utilisé dans la composition de l'invention est généralement introduit dans la phase grasse liquide de l'émulsion.

Par « polymère semi-cristallin », on entend au sens de l'invention, des polymères comportant une partie cristallisable, chaîne pendante ou séquence dans le squelette, et une partie amorphe dans le squelette et présentant une température de changement de phase réversible du premier ordre, en particulier de fusion (transition solide-liquide). Par « polymères », on entend au sens de l'invention, des composés comportant au moins 2 motifs répétitifs, de préférence au moins 3 motifs répétitifs et plus spécialement au moins 10 motifs répétitifs. Lorsque la partie cristallisable est une séquence du squelette polymérique, cette séquence cristallisable est de nature chimique différente de celle des séquences amorphes ; le polymère semi-cristallin est dans ce cas un polymère séquencé par exemple du type dibloc, tribloc ou multibloc.

De façon avantageuse, le ou les polymères semi-cristallins de la composition de l'invention ont une masse moléculaire moyenne en nombre Mn supérieure ou égale à 2000, allant par exemple de 2 000 à 800 000, de préférence de 3 000 à 500 000, par exemple de 4 000 à 150 000, et mieux de 4 000 à 99 000.

Dans la composition selon l'invention les polymères semi-cristallins sont avantageusement solubles dans la phase grasse à au moins 1 % en poids, à une température supérieure à leur température de fusion. En dehors des chaînes ou séquences cristallisables, les séquences des polymères sont amorphes. Par « chaîne ou séquence cristallisable », on entend au sens de l'invention une chaîne ou séquence qui, si elle était seule, passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitifs du polymère.

De préférence, le squelette polymérique des polymères semi-cristallins est soluble dans la phase grasse liquide.

De façon préférée, les polymères semi-cristallin utilisés dans la composition de l'invention présentent une température de fusion (ou point de fusion) pF inférieure à 70 °C, de préférence inférieure à 50 °C, cette température étant au moins égale à la température du support kératinique devant recevoir la composition selon l'invention. Le polymère semi-cristallin a une température de fusion pF telle que 25 °C ≤ pF< 70 °C et de préférence 30 °C ≤ pF < 50 °C. La température de fusion peut être mesurée notamment par toute méthode connue et en particulier avec un calorimètre à balayage différentiel (D.S.C).

De préférence, les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères séquencés ou multiséquencés. Ils peuvent être obtenus par polymérisation de monomères à doubles liaisons réactives (liaisons éthyléniques) ou par polycondensation. Lorsque les polymères de l'invention sont des polymères à chaînes latérales cristallisables, ceux-ci sont avantageusement sous forme aléatoire ou statistique.

De préférence, les polymères semi-cristallins de l'invention sont d'origine synthétique. En outre, ils ne comportent pas de squelette polysaccharidique.

Les polymères semi-cristallins décrits dans le présent texte sont en particulier :
1) les copolymères séquencés de polyoléfines à cristallisation contrôlée, dont les monomères sont décrits dans le document EP-A-951897 ;
2) les polycondensats et notamment les polycondensats polyesters, aliphatiques ou aromatiques, et les copolyesters aliphatiques/aromatiques ;
3) les homo- ou co-polymères portant au moins une chaîne latérale cristallisable et les homo- ou co-polymères portant dans le squelette au moins une séquence cristallisable, comme ceux décrits dans le document US-A-5,156,911 ;
4) les homo- ou co-polymères portant au moins une chaîne latérale cristallisable à groupement(s) fluoré(s), tels que décrits dans le document WO-A-01/19333 ;
5) et leurs mélanges.

Dans les deux derniers cas (3 et 4), la ou les chaînes latérales ou séquences cristallisables sont hydrophobes.

Les polymères cristallins à chaînes latérales cristallisables ou portant dans le squelette au moins une séquence cristallisable sont décrits ci-dessous.

### A) Polymères semi-cristallins à chaînes latérales cristallisables

On peut citer en particulier ceux définis dans les documents US-A-5,156,911 et WO-A-01/19333. Ce sont des homopolymères ou copolymères comportant de 50 à 100 % en poids de motifs résultant de la polymérisation d'un ou de plusieurs monomères porteurs de chaîne(s) latérale(s) hydrophobe(s) cristallisable(s).

Ces homo- ou co-polymères sont de toute nature du moment qu'ils présentent les conditions indiquées ci-après avec, en particulier, la caractéristique d'être solubles ou dispersables dans la phase grasse liquide par chauffage au-dessus de leur température de fusion pF. Ils peuvent résulter :
- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique ;
- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.
   a) D'une façon générale les motifs (chaînes ou séquences) cristallisables des polymères semi-cristallins décrits dans le texte, proviennent de monomère(s) à séquence(s) ou chaîne(s) cristallisable(s), utilisé(s) pour la fabrication des polymères semi-cristallins. Ces polymères sont choisis notamment parmi les homopolymères et copolymères résultant de la polymérisation d'au moins un monomère à chaîne(s) cristallisable(s) qui peut être représenté par la formule X : 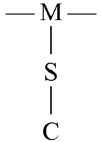 où M représente un atome du squelette polymérique, S représente un espaceur et C représente un groupe cristallisable.

Les chaînes « -S-C » cristallisables peuvent être aliphatiques ou aromatiques, éventuellement fluorées ou perfluorées. « S » représente notamment un groupe (CH₂)ₙ ou (CH₂CH₂O)ₙ ou (CH₂O), linéaire ou ramifié ou cyclique, n étant un nombre entier allant de 0 à 22. De préférence « S » est un groupe linéaire. De préférence, « S » et « C » sont différents.

Lorsque les chaînes cristallisables sont des chaînes aliphatiques (alkyle), elles comportent au moins 11 atomes de carbone et au plus 40 atomes de carbone et mieux au plus 24 atomes de carbone. Il s'agit notamment de chaînes alkyle possédant au moins 12 atomes de carbone, et de préférence, il s'agit de chaînes alkyle comportant de 14 à 24 atomes de carbone (C₁₄-C₂₄). Il peut s'agir de chaînes alkyle hydrocarbonées (atomes de carbone et d'hydrogène) ou chaînes alkyle fluorées ou perfluorées (atomes de carbone, atomes de fluor et éventuellement atomes d'hydrogène). Lorsqu'il s'agit de chaînes alkyle fluorées ou perfluorées, elles comportent au moins 11 atomes de carbone dont au moins 6 atomes de carbone sont fluorés.

Comme exemples de polymères ou copolymères semi-cristallins à chaîne(s) cristallisable(s), on peut citer ceux résultant de la polymérisation d'au moins un monomère à chaîne cristallisable choisi parmi les (méth)acrylates d'alkyle saturés en C₁₄-C₂₄ (C₁₄-C₂₄ signifie que le groupe alkyle comporte de 14 à 24 atomes de carbone) ; les (méth)acrylates de perfluoroalkyle en C₁₁-C₁₅ (groupe alkyle avec 11 à 15 atomes de carbone) ; les N-alkyl (méth)acrylamides en C₁₄ à C₂₄ avec ou sans atome de fluor (groupe alkyle avec 14 à 24 atomes de carbone) ; les esters vinyliques à chaînes alkyle ou perfluoroalkyle en C₁₄ à C₂₄ (groupe alkyle avec 14 à 24 atomes de carbone), avec une chaîne perfluoroalkyle comportant au moins 6 atomes de fluor ; les éthers vinyliques à chaînes alkyle ou perfluoroalkyle en C₁₄ à C₂₄ (groupe alkyle avec 14 à 24 atomes de carbone), avec une chaîne perfluoroalkyle comportant au moins 6 atomes de fluor ; les alpha-oléfines en C₁₄ à C₂₄ (groupe alkyle avec 14 à 24 atomes de carbone), comme par exemple l'octadécène ; les para-alkyl styrènes en C₁₄ à C₂₄ (groupe alkyle avec 14 à 24 atomes de carbone), leurs mélanges.

Par « alkyle », on entend au sens de l'invention un groupement saturé notamment comportant de 8 à 24 atomes de carbone (C₈ à C₂₄), sauf mention exprès.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères utilisés dans la composition de l'invention sont des copolymères, ils contiennent, en plus, de 0 à 50 % de groupes Y ou Z résultant de la copolymérisation :
α) avec Y qui est un monomère polaire ou non polaire ou un mélange des deux :
   - Lorsque Y est un monomère polaire, c'est soit un monomère porteur de groupes polyoxyalkylénés (notamment oxyéthylénés et/ou oxypropylénés), un (méth)acrylate d'hydroxyalkyle comme l'acrylate d'hydroxyéthyle, le (méth)acrylamide, un N-alkyl(méth)acrylamide, un NN-dialkyl(méth)acrylamide comme par exemple le NN-diisopropylacrylamide ou la N-vinyl-pyrrolidone (NVP), le N-vinyl-caprolactame, soit un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.
   - Lorsque Y est un monomère non polaire, il peut être un ester du type (méth)acrylate d'alkyle linéaire ramifié ou cyclique, un ester vinylique, un alkyl vinyl éther, une alpha-oléfine, le styrène ou le styrène substitué par un groupe alkyle comportant de 1 à 10 atomes de carbone (C₁ à C₁₀), comme l'α-méthylstyrène, un macromonomère du type polyorganosiloxane à insaturation vinylique.
β) avec Z qui est un monomère polaire ou un mélange de monomères polaires, Z ayant la même définition que le « Y polaire » défini ci-dessus.

De préférence, les polymères semi-cristallins à chaîne latérale cristallisable sont choisis parmi les homopolymères d'alkyl(méth)acrylate ou d'alkyl(méth)acrylamide avec un groupe alkyle tel que défini ci-dessus, et notamment en C₁₄-C₂₄ ; les copolymères de ces monomères avec un monomère hydrophile de préférence de nature différente de l'acide (méth)acrylique ; et leurs mélanges. Il peut s'agir par exemple comme copolymères, des copolymères d'alkyl(méth)acrylate ou d'alkyl (méth)acrylamide avec un groupe alkyle en C₁₄ à C₂₄, avec la N-vinylpyrrolidone, l'hydroxyéthyl (méth)acrylate ; ou leurs mélanges.

Les polymères semi-cristallins de la composition de l'invention peuvent être non réticulés ou réticulés en partie du moment que le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère, comme par exemple les interactions dipolaires entre ionomères carboxylates, ces interactions étant en faible quantité et portées par le squelette du polymère ; soit à une séparation de phase entre les séquences cristallisables et les séquences amorphes portées par le polymère.

De préférence, les polymères semi-cristallins de la composition selon l'invention sont non réticulés.

A titre d'exemple particulier de polymère semi-cristallin utilisable dans la composition selon l'invention, on peut citer les produits Intelimer® de la société Landec décrits dans la brochure « Intelimer® polymers ». Ces polymères sont sous forme solide à température ambiante (25 °C). Ils sont porteurs de chaînes latérales cristallisables et présentent le monomère de formule X précédente. On peut citer notamment le « Landec IP22 », ayant une température de fusion pF de 56 °C, qui est un produit visqueux à température ambiante, imperméable, non-collant.

On peut aussi mentionner les polymères semi-cristallins décrits dans les exemples 3, 4, 5, 7, 9 du document US-A-5,156,911, résultant de la copolymérisation d'acide acrylique et d'alkyl(méth)acrylate en C₅ à C₁₆ ayant un pF allant de 20 °C à 35 °C, et plus particulièrement ceux résultant de la copolymérisation :
. d'acide acrylique, d'hexadécylacrylate et d'isodécylacrylate dans un rapport 1/16/3,
. d'acide acrylique et de pentadécylacrylate dans un rapport 1/19,
. d'acide acrylique, d'hexadécylacrylate, d'éthylacrylate dans un rapport 2,5/76,5/20,
. d'acide acrylique, d'hexadécylacrylate et de méthylacrylate dans un rapport 5/85/10,
. d'acide acrylique, d'octadécylméthacrylate dans un rapport 2,5/97,5.

On peut aussi mentionner le polymère « Structure O » commercialisé par la société National Starch, tel que celui décrit dans le document US-A-5,736,125, de pF 44 °C, ainsi que les polymères semi-cristallins à chaînes pendantes cristallisables comportant des groupements fluorés tels que décrits dans les exemples 1, 4, 6, 7 et 8 du document WO-A-01/19333.

On peut encore mentionner les polymères semi-cristallins obtenus par copolymérisation d'acrylate de stéaryle et d'acide acrylique ou de NVP, tels que décrits dans le document US-A-5,519,063 ou EP-A- 0550745, et plus spécialement ceux décrits dans les exemples 1 et 2, ci-après, de préparation de polymère, de température de fusion respectivement de 40 °C et 38 °C.

On peut aussi mentionner les polymères semi-cristallins obtenus par copolymérisation de l'acrylate de béhényle et de l'acide acrylique ou de NVP, tels que décrits dans les documents US-A-5,519,063 et EP-A- 0 550745, et plus spécialement ceux décrits dans les exemples 3 et 4, ci-après, de préparation de polymère, de température de fusion respectivement de 60 °C et 58 °C.

Selon un mode particulier de réalisation de l'invention, les polymères semi-cristallins utilisés ne comportent pas de groupement carboxylique.

La quantité de polymère semi-cristallin dans la composition de l'invention peut aller par exemple de 0,1 à 30 % en poids de matière active, de préférence de 0,5 à 20 % en poids de matière active et mieux de 1 à 10 % en poids de matière active, et préférentiellement de 1 à 5 % en poids par rapport au poids total de la composition.

En particulier, le polymère semi-cristallin et le tensioactif géminé peuvent être présents dans une composition selon l'invention dans un rapport pondéral polymère(s) semi-cristallin(s)/tensioactif(s) géminé(s) allant de 1 à 10, notamment allant de 1,5 à 9.

### Phase grasse

Comme précisé précédemment, la composition est une émulsion huile-dans-eau dont la phase grasse peut aller de 10 à 50 % en poids, en particulier de 15 à 45 % en poids et de préférence de 20 à 40 % en poids par rapport au poids total de la composition.

Au sens de l'invention, la phase grasse inclut tout corps gras liquides, généralement des huiles, ou solides à l'image des cires ou composés pâteux présents dans ladite composition.

Cette phase grasse contient au moins 40 % en poids d'huile(s) non volatile(s) telle(s) que décrite(s) ci-dessous.

On entend par huile, tout corps gras sous forme liquide à température ambiante (25 °C) et à pression atmosphérique.

La ou les huiles peuvent être présentes à raison de 0,1 à 50 % en poids, en particulier d'au moins 5 à 40 % en poids, par rapport au poids total de la composition cosmétique selon l'invention.

Les huiles volatiles ou non volatiles peuvent être des huiles hydrocarbonées notamment d'origine animale ou végétale, des huiles synthétiques, des huiles siliconées, des huiles fluorées, ou leurs mélanges.

Au sens de la présente invention, on entend par « huile siliconée », une huile comprenant au moins un atome de silicium, et notamment au moins un groupe Si-O.

On entend par « huile hydrocarbonée », une huile contenant principalement des atomes d'hydrogène et de carbone et éventuellement des atomes d'oxygène, d'azote, de soufre et/ou de phosphore.

### Huiles non volatiles

Au sens de la présente invention, on entend par « huile non-volatile », une huile ayant une pression de vapeur inférieure à 0,13 Pa (0,01 mm de Hg).

Les huiles non volatiles peuvent notamment être choisies parmi les huiles hydrocarbonées le cas échéant fluorées et/ou les huiles siliconées non volatiles.

Comme huile hydrocarbonée non volatile convenant à la mise en oeuvre de l'invention, on peut notamment citer :
- les huiles hydrocarbonées d'origine animale,
- les huiles hydrocarbonées d'origine végétale telles que les esters de phytostéaryle, tels que l'oléate de phytostéaryle, l'isostéarate de physostéaryle et le glutanate de lauroyl/octyldodécyle/phytostéaryle, par exemple vendu sous la dénomination ELDEW PS203 par AJINOMOTO, les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides héptanoïques ou octanoïques, les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STEARINERIES DUBOIS ou ceux vendus sous les dénominations MIGLYOL 810^{®}, 812^{®} et 818^{®} par la société DYNAMIT NOBEL,
- les huiles hydrocarbonées d'origine minérale ou synthétique comme par exemple :
   - les éthers de synthèse ayant de 10 à 40 atomes de carbone ;
   - les hydrocarbures linéaires ou ramifiés, d'origine minérale ou synthétique tels que la vaseline, les polydécènes, le polyisobutène hydrogéné tel que le parléam, le squalane et leurs mélanges, et en particulier le polyisobutène hydrogéné,
   - les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10.

Les esters peuvent être notamment choisis parmi les esters, notamment d'acide gras comme par exemple :
❖ l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en C₁₂ à C₁₅, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle ;
❖ les esters de polyols, et les esters de pentaétrythritol, comme le tétrahydroxystéarate/tétraisostéarate de dipentaérythritol,
❖ les esters de dimères diols et dimères diacides tels que les Lusplan DD-DA5^{®} et Lusplan DD-DA7^{®}, commercialisés par la société NIPPON FINE CHEMICAL et décrits dans la demande FR 03 02809,
   - les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol,
   - les acides gras supérieurs tels que l'acide oléique, l'acide linoléique, l'acide linolénique et leurs mélanges, et
   - les carbonates de di-alkyle, les 2 chaînes alkyles pouvant être identiques ou différentes, tel que le dicaprylyl carbonate commercialisé sous la dénomination Cetiol CC®, par Cognis,
   - les huiles de silicone non volatiles, comme par exemple les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 Cst, et leurs mélanges,
      - et leurs mélanges.

Les huiles non volatiles peuvent être également choisies parmi les filtres organiques liquides tels que ceux cités ci-après parmi la liste de filtres UV organiques.

Les huiles non volatiles peuvent être présentes dans une composition selon l'invention en une teneur allant de 1 % à 40 % en poids, notamment de 3 % à 20 % en poids, par rapport au poids total de la composition.

### Huiles volatiles

Une composition conforme à l'invention peut également comprendre au moins une huile volatile.

Au sens de la présente invention, on entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm Hg), en particulier allant de 1,3 Pa à 13 000 Pa (0,01 à 100 mm Hg), et plus particulièrement allant de 1,3 Pa à 1300 Pa (0,01 à 10 mm Hg).

Les huiles hydrocarbonées volatiles peuvent être choisies parmi les huiles hydrocarbonées ayant de 8 à 16 atomes de carbone, et notamment les alcanes ramifiés en C₈-C₁₆ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars^{®} ou de Permethyls^{®}.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées telles que le nonafluorométhoxybutane ou le perfluorométhylcyclopentane, et leurs mélanges.

Il est également possible d'utiliser un mélange des huiles précédemment citées.

Selon une variante de réalisation, les compositions selon l'invention contient au moins 5 % en poids voire au moins 10 % en poids d'huile(s) non siliconée(s).

Les autres corps gras pouvant être présents dans la phase huileuse sont par exemple les acides gras comportant de 8 à 30 atomes de carbone, comme l'acide stéarique, l'acide laurique, l'acide palmitique ; les alcools gras comportant de 8 à 30 atomes de carbone, comme l'alcool stéarylique, l'alcool cétylique et leurs mélanges (alcool cétéarylique).

La phase grasse liquide peut, également, contenir outre des huiles, d'autres composés solubilisés dans les huiles tels que des agents gélifiants et/ou structurants.

Ces composés peuvent notamment être choisis parmi les gommes telles que les gommes de silicone (diméthiconol) ; les résines de silicone telles que la trifluorométhyl-C1-4-alkyldimethicone et la Trifluoropropyldimethicone, et les élastomères de silicone comme les produits commercialisés sous les dénominations « KSG » par la société Shin-Etsu, sous la dénomination « Trefil » par la société Dow Corning ou sous les dénominations « Gransil » par la société Grant Industries ; et leurs mélanges.

### Corpus gras solides

La composition selon l'invention peut également comprendre au moins un corps gras solide choisi parmi les cires, les corps gras pâteux, et leurs mélanges.

La cire est solide à température ambiante (25 °C), à changement d'état solide/liquide réversible, ayant une température de fusion supérieure à 30 °C pouvant aller jusqu'à 200 °C, une dureté supérieure à 0,5 MPa et présentant à l'état solide une organisation cristalline anisotrope.

Elle peut être hydrocarbonée, fluorée et/ou siliconée et être d'origine animale, végétale, minérale ou synthétique.

Elle peut être choisie par exemple parmi la cire d'abeille, la cire de Carnauba, la cire de Candelilla, les cires de paraffine, l'huile de ricin hydrogénée, les cires synthétiques comme les cires de polyéthylène (de préférence de poids moléculaire compris entre 400 et 600) ,les cires de Fischer-Tropsch, les cires de silicone comme les alkyl- ou alkoxy-diméthicone ayant de 16 à 45 atomes de carbone, les cérésines ou les ozokérites, comme par exemple les isoparaffines dont le point de fusion est inférieur à 40 °C, tel que l'EMW-0003, commercialisé par la société NIPPON SEIROU, les oligomères d'α-oléfine, tel que les polymères PERFORMA V^{®} 825, 103 et 260, commercialisés par la société NEW PHASE TECHNOLOGIES ; les copolymères éthylène-propylène, tel que le PERFORMALENE^{®} EP 700, et les cires microcristallines dont le point de fusion est supérieur à 85 °C, tel que les HI-MIC^{®} 1070, 1080, 1090 et 3080, commercialisées par NIPPON SEIROU, et leurs mélanges.

Selon un mode particulier de mise en oeuvre, la ou les cires utilisées dans les compositions cosmétiques conformes à la présente invention peut ou peuvent être présentes en une teneur variant d'environ 1 à environ 20 %, en particulier d'environ 2 à environ 10 %, par rapport au poids total de la composition.

Une composition cosmétique conforme à la présente invention peut également comprendre au moins un composé pâteux.

Par « pâteux » au sens de la présente invention, on entend un composé gras à changement d'état solide/liquide réversible et comportant à la température de 23 °C une fraction liquide et une fraction solide. On entend également par pâteux, le polylaurate de vinyle.

Un composé pâteux au sens de l'invention peut présenter avantageusement une dureté à 20 °C allant de 0,001 à 0,5 MPa, de préférence de 0,002 à 0,4 MPa.

Parmi les composés pâteux susceptibles d'être utilisés dans la composition selon l'invention, on peut citer la vaseline, le beurre de karité, le beurre de cacao, le beurre de shorea, les lanolines et les dérivés de lanoline comme les lanolines acétylées, les lanolines oxypropylénées ou le lanolate d'isopropyle, et leurs mélanges. On peut également utiliser des esters d'acides ou d'alcools gras, notamment ceux ayant 20 à 65 atomes de carbone comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le « THIXINR^{®} » de Rheox.

On peut également citer les polyesters résultant de l'estérification d'un acide carboxylique et d'un ester acide hydroxycarboxylique aliphatique. Par exemple, le RISOCAST^{®} DA-L (ester issu de la réaction d'estérification de l'huile de ricin hydrogéné avec de l'acide dilinoléïque dans des proportions de 2 pour 1) et le RISOCAST^{®} DA-H (ester résultant de l'estérification de l'huile de ricin hydrogénée avec de l'acide isostéarique dans des proportions de 4 pour 3) commercialisés par la société japonaise KOKYU ALCOHOL KOGYO.

On peut aussi citer les composés pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55 °C, comme les stéaryl diméthicones notamment ceux vendus par la société DOW CORNING sous les noms commerciaux de DC2503^{®} et DC25514^{®} et leurs mélanges.

La composition peut également comprendre un additif cosmétique notamment choisi parmi les charges, les tensioactifs, les actifs cosmétiques ou dermatologiques, les filtres UV, les polymères filmogènes, les gélifiants, les conservateurs, les parfums, les pigments, les actifs comme les vitamines, les extraits de plantes.

Comme précisé précédemment, les compositions selon l'invention sont particulièrement avantageuses pour formuler des filtres organiques.

### Filtres UV organiques (ou filtres solaires)

Ainsi, selon une variante de réalisation, les compositions selon l'invention contiennent un ou plusieurs filtres organiques.

Plus précisément, une composition de l'invention peut contenir au moins un filtre UV organique choisi parmi les filtres organiques hydrophiles, les filtres organiques lipophiles et leurs mélanges. Selon un mode particulier de réalisation de l'invention, on peut y associer un ou plusieurs filtres physiques.

Comme exemples de filtres organiques, actifs dans l'UV-A et/ou l'UV-B, pouvant être utilisés dans la composition de l'invention, on peut citer par exemple, désignés ci-dessus sous leur nom CTFA :

### Dérivés de l'acide para-aminobenzoique (PABA) :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA, (liquide)
- PEG-25 PABA vendu sous le nom « UVINUL P25 » par BASF,

### Dérivés salicylique :

- Homosalate vendu sous le nom « Eusolex HMS » par Rona/EM Industries, (liquide)
- Ethylhexyl Salicylate (ou salicylate d'éthyl hexyle) vendu sous le nom « NEO HELIOPAN OS » par Haarmann et REIMER, (liquide)
- Dipropyleneglycol Salicylate vendu sous le nom « DIPSAL » par SCHER,
- TEA Salicylate, vendu sous le nom « NEO HELIOPAN TS » par Haarmann et REIMER, (liquide)

### Dérivés du dibenzoylméthane :

- Butyl Methoxydibenzoylmethane vendu notamment sous le nom commercial « PARSOL 1789 » par HOFFMANN LA ROCHE, (liquide)
- Isopropyl Dibenzoylmethane,

### Dérivés cinnamiques :

- Ethylhexyl Methoxycinnamate (ou Octyl Methoxycinnamate) vendu notamment sous le nom commercial « PARSOL MCX » par HOFFMANN LA ROCHE,
- Isopropyl Methoxycinnamate,
- Isoamyl Methoxycinnamate vendu sous le nom commercial « NEO HELIOPAN E 1000 » par HAARMANN et REIMER, (liquide)
- Cinoxate,
- DEA Methoxycinnamate, (liquide)
- Diisopropyl Methylcinnamate, (liquide)
- Glyceryl Ethylhexanoate Dimethoxycinnamate

### Dérivés de β,β-diphénylacrylate :

- Octocrylene (α-cyano-β,β-diphénylacrylate de 2-éthylhexyle) vendu notamment sous le nom commercial « UVINUL N539 » par BASF, (liquide)
- Etocrylene, vendu notamment sous le nom commercial « UVINUL N35 » par BASF,

### Dérivés de la benzophénone :

- Benzophenone-1 vendu sous le nom commercial «UVINUL 400 » par BASF,
- Benzophenone-2 vendu sous le nom commercial « UVINUL D50 » par BASF,
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « Helisorb 11 » par Norquay
- Benzophenone-8 vendu sous le nom commercial « Spectra-Sorb UV-24 » par American Cyanamid
- Benzophenone-9 vendu sous le nom commercial « UVINUL DS-49 » par BASF,
- Benzophenone-12

### Dérivés du benzylidène camphre :

- 3-Benzylidene camphor fabriqué sous le nom « MEXORYL SD » par CHIMEX,
- 4-Methylbenzylidene camphor vendu sous le nom « EUSOLEX 6300 » par MERCK,
- Benzylidene Camphor Sulfonic Acid fabriqué sous le nom « MEXORYL SL » par CHIMEX,
- Camphor Benzalkonium Methosulfate fabriqué sous le nom « MEXORYL SO » par CHIMEX,
- Terephthalylidene Dicamphor Sulfonic Acid fabriqué sous le nom « MEXORYL SX » par CHIMEX,
- Polyacrylamidomethyl Benzylidene Camphor fabriqué sous le nom « MEXORYL SW » par CHIMEX,

### Dérivés du phenyl benzimidazole :

- Phenylbenzimidazole Sulfonic Acid vendu notamment sous le nom commercial « EUSOLEX 232 » par MERCK,
- Benzimidazilate vendu sous le nom commercial « NEO HELIOPAN AP » par Haarmann et REIMER,

### Dérivés de la triazine :

- Anisotriazine vendu sous le nom commercial « TINOSORB S » par CIBA GEIGY,
- Ethylhexyl triazone vendu notamment sous le nom commercial « UVINUL T150 » par BASF,
- Diethylhexyl Butamido Triazone vendu sous le nom commercial « UVASORB HEB » par SIGMA 3V,

### Dérivés du phenyl benzotriazole :

- Drometrizole Trisiloxane vendu sous le nom « Silatrizole » par RHODIA CHIMIE,

### Dérivés anthraniliques :

- Menthyl anthranilate vendu sous le nom commercial « NEO HELIOPAN MA » par Haarmann et REIMER, (liquide)

### Dérivés d'imidazolines :

- Ethylhexyl Dimethoxybenzylidene Dioxoimidazoline Propionate,

### Dérivés du benzalmalonate :

- Polyorganosiloxane à fonctions benzalmalonate vendu sous la dénomination commerciale « PARSOL SLX » par HOFFMANN LA ROCHE
- et leurs mélanges.

Les filtres UV organiques plus particulièrement préférés sont choisis parmi les composés suivants :
- Ethylhexyl salicylate,
- Ethylhexyl triazone,
- Butyl Methoxydibenzoylmethane,
- Ethylhexyl Methoxycinnamate,
- Octocrylène,
- Phenylbenzimidazole Sulfonic Acid (acide phenylbenzimidazole sulfonique),
- Terephthalylidene Dicamphor Sulfonic Acid (acide téréphthalylidène dicamphosulfonique),
- Benzophenone-3,
- Benzophenone-4,
- Benzophenone-5,
- 4-Methylbenzylidene camphor (4-méthylbenzylidène camphre),
- Benzimidazilate,
- Anisotriazine,
- Ethylhexyl triazone,
- Diethylhexyl Butamido Triazone,
- Methylene bis-Benzotriazolyl Tetramethylbutylphenol (méthylène bis-benzotriazolyl tétraméthylbutylphenol),
- Drometrizole Trisiloxane,
- et leurs mélanges.

Le ou les filtres organiques peuvent être présents en une quantité allant de 0,1 à 25 % en poids, de préférence de 1 à 20 % en poids, et mieux 5 à 15 % en poids par rapport au poids total de la composition.

Comme filtres physiques pouvant être également présents dans les compositions de l'invention, on peut citer par exemple les pigments et nano-pigments d'oxydes métalliques, enrobés ou non enrobés, notamment les oxydes de titane, de fer, de zirconium, de zinc ou de cérium, et leurs mélanges, ces oxydes pouvant être sous forme de micro- ou nanoparticules (nanopigments), éventuellement enrobées.

Bien entendu l'homme du métier veillera à choisir ce ou ces éventuels composés complémentaires, et/ou leur quantité, de manière telle que les propriétés avantageuses des composés selon l'invention ne soient pas, ou substantiellement pas, altérées par l'adjonction envisagée.

Les compositions peuvent être plus ou moins fluide et avoir l'aspect d'une crème blanche ou colorée, d'une pommade, d'un lait, d'une lotion, d'une brume, d'un sérum, d'une pâte, d'une mousse.

Selon une variante de réalisation, elles peuvent se présenter sous la forme d'un produit de maquillage tel que par exemple un fond de teint.

Elles peuvent également se présenter avantageusement sous la forme de produits solaires.

Les exemples figurant ci-après sont présentés à titre illustratif et non limitatif du domaine de l'invention.
- Le tensioactif géminé mis en oeuvre dans les exemples ci-après est un mélange d'alcool béhénylique (Behenyl Alcohol), de stéarate de glycéryle (Glyceryl Stearate), de stéarate de monocitrate de glycéryle (Glyceryl Stearate Citrate) et de Sodium Dicocoyléthylènediamine PEG-15 Sulfate commercialisé par la société SASOC sous le nom Ceralution ^{®} H.
- Le polymère semi-cristallin utilisé est un polyacrylate de stéaryle commercialisé par la société LANDEC sous le nom Intelimer IPA 13-1..

### Exemple 1

Une composition cosmétique conforme à l'invention et dont la formulation détaillée est soumise ci-après dans le tableau I est préparée et ses propriétés en terme de stabilité et de qualité sensorielle sont testées.

Sa stabilité dans le temps est appréciée à l'issu d'un stockage de celle-ci au repos pendant 2 mois à une température respectivement de 37 °C puis de 45 °C. A titre comparatif, deux autres formulations ont été préparées dans lesquelles le tensioactif géminé est remplacé par soit un mélange de cétylstéarylglucoside et d'alcools cétylique, stéaryle 12/46/42 commercialisé par SEPPIC sous la dénomination MONTANOV^{®} (Témoin 1), soit du distéarate de polyglycéryl-3 méthylglucose commercialisé par Goldschmidt sous la dénomination Tegocare 450^{®}. (Témoin 2)

Les formulations correspondantes figurent également dans le tableau I ci-après.

**Tableau 1**

| Nom chimique | Formule selon l'invention | Formule témoin 1 | Formule témoin 2 |
|---|---|---|---|
| Tensio actif géminé à 15 % MA Ceralution ^{®}H de la société SASOL | 3,5 soit 0,525 MA | - | - |
| Mélange de cetylstearyl glucoside et d'alcools cetylique, stearylique (12/46/42) (MONTANOV 68^{®} de chez Seppic) | - | 3,5 | - |
| Mélange de mono-di-stéarate de méthyl glucose et de stéarate de polyglycerine-3 (TEGO CARE 450^{®} de chez Goldschmidt) | - | - | 2,5 |
| Laurate d'héxyl-2-décyle | 4 | 4 | 4 |
| Isohexadécane | 8 | 8 | 8 |
| Cyclohéxadiméthylsilo xane | 2,5 | 2,5 | 2,5 |
| Glycérine | 7 | 7 | 7 |
| Acide phényl-2 benzimidazol sulfonique-5 | 1,7 | 1,7 | 1,7 |
| P-methoxy-4-cinnamate d'éthyl-2 hexyle | 7,5 | 7,5 | 7,5 |
| Polyacrylate de stéaryle (Intelimer IPA 13-1^{®} de chez Landec) | 1 | 1 | 1 |
| Parfum | 0,5 | 0,5 | 0,5 |
| conservateurs | 0,4 | 0,4 | 0,4 |
| Eau | Qsp 100 % | Qsp 100 % | Qsp 100 % |
| Acide polyacrylamidométhylpropane sulfonique neutralise partiellement à l'ammoniac (Hostacerin AMPS^{®} de Clariant) | 0,5 | 0,5 | 0,5 |
| Gomme Xanthane | 0,25 | 0,25 | 0,25 |
| Acide terephtalydidène-3,3'-dicamphosulfonique 10-10' dans l'eau à 33 % non stabilisé | 2,12 | 2,12 | 2,12 |
| Soude | Qsp pH 6.5 | Qsp pH 6.5 | Qsp pH 6.5 |
| Silice (SB 700^{®} de Miyoshi Kasei) | 3 | 3 | 3 |

### Résultats :

| | Formule selon l'invention | Formule témoin 1 | Formule témoin 2 |
|---|---|---|---|
| Stabilité | Satisfaisante | Non stable | Non stable |
| Propriétés cosmétiques | Toucher doux, non gras, léger | Texture hétérogène, grasse, collante « savonneuse » (effet blanchissant) | Texture hétérogène, grasse, collante « savonneuse » (effet blanchissant) |

En terme de stabilité, seule l'émulsion conforme à la présente invention est satisfaisante. L'examen au microscope permet d'observer une émulsion fine et régulière par opposition aux formulations témoins dont les émulsions sont plus grossières, plus irrégulières. Ces mêmes résultats sont confirmés par évaluation macroscopique. Les formulations témoins présentent, après deux mois de conservation que ce soit à 37 °C ou à 45 °C une texture hétérogène avec des amas visibles à l'oeil nu. Seule la formulation selon l'invention demeure lisse et fine.

L'association selon l'invention assure manifestement une meilleure dispersion des charges de silice et prévient efficacement leur agglomération.

### Exemple 2

### Crème hydratante

| | % |
|---|---|
| Phase huileuse | |
| Tensio actif géminé à 15 % MA (Ceralution ^{®}H de la société SASOL) | 5 soit 0,75 % MA |
| Isoparaffine hydrogénée (Parleam^{®} de chez Nof Corporation) | 10 |
| Cire de polyméthylène (Cirebelle 303^{®} de chez Sasol) | 4 |
| Mélange de poly dimethylsiloxane alpha-omega dihydroxyle / poly dimethylsiloxane 5 cst (Dow Corning 1503 fluid^{®} de chez Dow Corning) | 7,5 |
| Pentaoctanoate de pentaeritrytyle | 5 |
| Polydécene hydrogéné (SILKFLO 366 NF POLYDECENE^{®} de chez Ineos) | 5 |
| Polyacrylate de stéaryle (Intelimer IPA 13-1^{®} de chez Landec) | 2 |

| Phase aqueuse | |
|---|---|
| Acide poly acrylamidométhyl propane sulfonique neutralisé partiellement à l'ammoniaque et hautement réticulé (Hostacerin AMPS^{®} de chez Clariant) | 0,5 |
| Conservateurs | Qs |
| Glycérine | 7 |
| Eau | Qsp 100 % |

| Phase poudres | |
|---|---|
| Microsphères de silice (SB 700^{®} de Miyoshi Kasei) | 3 |

### Mode opératoire

Après homogénéisation des différentes phases, on disperse de la phase huileuse dans la phase aqueuse sous agitation à 75 °C. On obtient une crème ferme, fondante à l'application apportant nutrition intense et hydratation sans film gras désagréable. La composition est également stable après stockage 2 mois à 25 °C et 37 °C.

### Exemple 3

En tableau 2, figure la composition d'une formulation conforme à l'invention. Ce tableau rend également compte d'une formule témoin dans laquelle le polyacrylate de stéaryle est remplacé par de la cire d'abeille.

**Tableau 2**

| Nom chimique | Formule selon l'invention | Formule témoin |
|---|---|---|
| Tensio actif géminé à 15 % MA (Ceralution^{®} H de la société SASOL) | 3,5 soit 0,525 MA | 3,5 soit 0,525 MA |
| Cyclohéxadiméthylsilo xane | 2,5 | 2,5 |
| Glycérine | 7 | 7 |
| 4-tertiobutyl-4'méthoxy-dibenzoylméthane | 3 | 3 |
| 2-cyano-3,3-diphénylacrylate de éthyl-2 hexyle | 7 | 7 |
| Salicylate d'éthyl-2 hexyle | 5 | 5 |
| Polyacrylate de stéaryle (Intelimer IPA 13-1^{®} de chez Landec) | 2 | - |
| Cire d'abeille | - | 2 |
| Parfum | 0,1 | 0,1 |
| conservateurs | 0,5 | 0,5 |
| Caprylyl glycol | 0,5 | 0,5 |
| Eau | Qsp 100 % | Qsp 100 % |
| mélange poly dimethylsiloxane alpha-omega dihydroxyle / cyclopenta dimethylsiloxane (14.7/85.3) (DOW CORNING 1501 FL^{®} de chez Dow Corning) | 2 | 2 |
| (Acide polyacrylamidométhylpropane sulfonique neutralise partiellement à l'ammoniac) (Hostacerin AMPS^{®} de Clariant) | 0,5 | 0,5 |
| Gomme Xanthane | 0,25 | 0,25 |
| Sel disodique de l'acide éthylène diamine tétracétique | 0,1 | 0,1 |
| Isononanoate d'isononyle | 4 | 4 |
| Copolymère styrène/acrylate (Sunspheres Powder^{®} de chez Rohm & Haas) | 3 | 3 |

Chaque composition a été conservée pendant 2 mois à des températures de 25 °C, 27 °C et 45 °C.

L'examen à l'oeil nu montre que la composition selon l'invention est homogène et lisse, tandis que la composition teneur présente des amas et est donc hétérogène, ce qui démontre que la présence de polyacrylate de stéaryle permet d'obtenir une composition stable.

L'examen au microscope après 2 mois de stockage à 45 °C montre que la composition selon l'invention est fine tandis que la composition témoin présente des zones des lachages des gouttes d'huiles et donc une hétérogénéité.

Par ailleurs, la composition appliquée sur la peau ne confère pas de sensation grasse, ni d'effet brillant.

## Revendications

1. Composition cosmétique ou dermatologique de type émulsion huile-dans-eau comprenant dans un milieu physiologiquement acceptable au moins un tensioactif géminé de formule (I) : 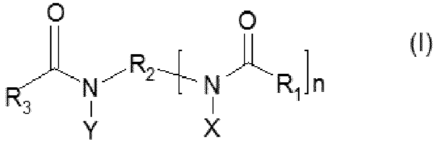 dans laquelle :
- R₁ et R₃ désignent, indépendamment l'un de l'autre, un radical alkyle ayant de 1 à 25 atomes de carbone ;
- R₂ désigne un espaceur constitué d'une chaîne alkylène linéaire ou ramifiée ayant de 1 à 12 atomes de carbone ;
- X et Y désignent, indépendamment l'un de l'autre, un groupement - (C₂H₄O)ₐ-(C₃H₆O)_{b}Z où
• Z désigne un atome d'hydrogène ou un radical -CH₂-COOM, -SO₃M,-P(O)(OM)₂, -C₂H₄-SO₃M, -C₃H₆-SO₃M ou -CH₂(CHOH)₄CH₂OH, où M représentent H ou un ion alcalin ou alcalino-terreux ou ammonium ou alcanolammonium,
• a va de 0 à 15,
• b va de 0 à 10, et
• la somme de a + b va de 1 à 25 ; et
- n va de 1 à 10,
en association avec une quantité efficace d'au moins un polymère semi-cristallin solide à température ambiante et ayant une température de fusion inférieure à 70 °C, comportant a) un squelette polymérique et b) au moins une chaîne organique latérale cristallisable et/ou une séquence organique cristallisable faisant partie du squelette dudit polymère, ledit polymère ayant une masse moléculaire moyenne en nombre supérieure ou égale à 2 000,
ledit polymère semi-cristallin étant choisi parmi les homopolymères et copolymères résultant de la polymérisation d'au moins un monomère à chaîne cristallisable choisi parmi les (méth)acrylates d'alkyle saturés en C₁₄-C₂₄ ;
ladite émulsion possédant un taux de phase grasse supérieur à 10 % en poids par rapport au poids total de la composition et comprenant plus de 40 % en poids d'huile(s) non volatile(s) par rapport au poids de la phase grasse ;
ladite composition étant différentes des compositions A et B suivantes :
| **Ingrédients** | **Composition A** (% en poids) | **Composition B** (% en poids) |
|---|---|---|
| EDTA disodique | 0,1 | 0,1 |
| Conservateur | 1,25 | 1,25 |
| Glycérine | 4 | 4 |
| Propyleneglycol | 4 | 4 |
| Eau | 61,65 | 61,65 |
| Dispersion de particules à 86% en poids Styrene/Acrylates Copolymer (Sunspheres Powder de ROHM & HAAS) | 2 | - |
| Poly(C₁₀-C₃₀)alkylacrylates (Intelimer 13-1 de LANDEC) | 1 | 1 |
| Mélange de séarate de glycéryle, alcool béhénique, sodium sulfate de dicocoylethylendiamine PEG-15, stéarate citrate de glycéryle | 2 | 2 |
| Isononyl isononaoate | 4 | 4 |
| Alcool cétylique | 0,5 | 0,5 |
| Butylmethoxy Dibenzoylmethane | 3 | 3 |
| Ethylhexyl Salicylate | 5 | 5 |
| Octocrylene | 7 | 7 |
| Parfum | 0,4 | 0,4 |
| Tocophérol | 0,2 | 0,2 |
| Acrylates Copolymer | 0,7 | 0,7 |
| Triéthanolamine | 0,2 | 0,2 |

2. Composition selon la revendication 1, **caractérisée par le fait que** chacun des groupements R₁-CO- et R₃-CO- désigne un reste d'acides gras de coco.

3. Composition selon l'une des revendications précédentes, **caractérisée par le fait que** pour le tensioactif géminé de formule (I), pour chacun des radicaux X et Y, la somme de a et b a une valeur moyenne allant de 10 à 20.

4. Composition selon l'une quelconque des revendications précédentes, **caractérisée par le fait que** pour le tensioactif géminé de formule (I) Y est le groupement-SO₃M où M est un ion alcalin tel qu'un ion sodium.

5. Composition selon l'une quelconque des revendications précédentes, **caractérisée par le fait que** pour le tensioactif géminé de formule (I), n est égal à 1.

6. Composition selon l'une quelconque des revendications précédentes, **caractérisée par le fait que** le tensioactif de formule (I) a la structure suivante :

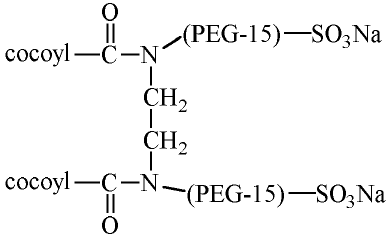

7. Composition selon l'une quelconque des revendications précédentes, **caractérisé par le fait que** le tensioactif géminé est mélangé à (a) un ester d'acide gras en C₆-C₂₂ et de glycéryle, (b) un diester d'acide gras en C₆-C₂₂ et d'acide citrique et de glycérol, et (c) un alcool gras en C₁₀-C₃₀.

8. Composition selon l'une quelconque des revendications précédentes, **caractérisée par le fait que** le tensioactif géminé est présent en une teneur allant de 0,01 à 5 % en poids, par rapport au poids total de la composition, de préférence allant de 0,1 à 3 % en poids, et préférentiellement allant de 0,2 à 1,5 % en poids.

9. Composition selon l'une quelconque des revendications précédentes, dans laquelle le polymère a une masse moléculaire moyenne en nombre allant de 3 000 à 500 000 et mieux de 4 000 à 99 000.

10. Composition selon l'une quelconque des revendications précédentes, **caractérisée en ce que** le polymère est soluble dans la phase grasse à au moins 1 % en poids à une température supérieure à sa température de fusion.

11. Composition selon l'une quelconque des revendications précédentes, **caractérisée en ce que** le polymère a une température de fusion pF telle que 30 °C ≤ pF < 50 °C.

12. Composition selon l'une quelconque des revendications précédentes contenant au moins un filtre organique.

13. Procédé de traitement cosmétique d'une matière kératinique, telle que la peau, du cuir chevelu, des cheveux, des cils, des sourcils, des ongles ou des muqueuses, **caractérisé par le fait qu'**on applique sur ladite matière kératinique, une composition selon l'une quelconque des revendications 1 à 12.

## Patentansprüche

1. Kosmetische oder dermatologische Zusammensetzung vom Typ Öl-in-Wasser-Emulsion umfassend, in einem physiologisch verträglichen Medium, mindestens ein Tensid-Paar der Formel (I): 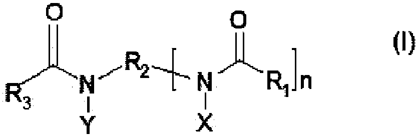 wobei
- R₁ und R₃, unabhängig voneinander, einen Alkylrest mit 1 bis 25 Kohlenstoffatomen bezeichnen;
- R₂ einen Spacer bezeichnet, der aus einer linearen oder verzweigten Alkylenkette mit 1 bis 12 Kohlenstoffatomen besteht;
- X und Y, unabhängig voneinander, eine Einheit -(C₂H₄O)ₐ-(C₃H₆P)_{b}Z bezeichnen, wobei
• Z ein Wasserstoffatom oder einen Rest -CH₂-COOM, -SO₃M, -P(O)(OM)₂,-C₂H₄-SO₃M, -C₃H₆-SO₃M oder -CH₂(CHOH)₄CH₂OH bezeichnet, wobei M H oder ein Alkalimetall- oder Erdalkalimetallion oder Ammonium oder Alkanolammonium bezeichnet,
• a 0-15 ist,
• b 0-10 ist, und
• die Summe von a + b 1-25 ist; und
- n 1-10 ist,
in Kombination mit einer wirksamen Menge von mindestens einem semikristallinen bei Umgebungstemperatur festen Polymer und mit einer Schmelztemperatur von kleiner als 70 °C, umfassend a) ein polymeres Grundgerüst und b) mindestens eine kristallisierbare organische Seitenkette und/oder eine kristallisierbare organische Sequenz, die Teil des Grundgerüsts des Polymers ist, wobei das Polymer ein durchschnittliches Zahlenmittelmolekulargewicht von größer oder gleich 2.000 aufweist, wobei das semikristalline Polymer ausgewählt ist aus Homo- und Copolymeren, die sich aus der Polymerisation von mindestens einem Monomer mit kristallisierbarer Kette ergeben, das ausgewählt ist aus gesättigten C₁₄-C₂₄-Alkyl-(meth)acrylaten;
wobei die Emulsion einen Gehalt an Fettphase von größer als 10 Gew.-%, bezogen auf das Gesamtgewicht der Zusammensetzung, besitzt und mehr als 40 Gew.-% nichtflüchtige(s) Öl(e), bezogen auf das Gewicht der Fettphase, umfasst;
wobei die Zusammensetzung von den folgenden Zusammensetzungen A und B verschieden ist:
| **Bestandteile** | **Zusammensetzung A** | **Zusammensetzung B** |
|---|---|---|
| | (Gew.-%) | (Gew.-%) |
| Di-Natrium-EDTA | 0,1 | 0,1 |
| Konservierungsstoff | 1,25 | 1,25 |
| Glycerin | 4 | 4 |
| Propylenglycol | 4 | 4 |
| Wasser | 61,65 | 61,65 |
| Dispersion von Teilchen mit 86 Gew.-% Styrol/Acrylate-Copolymer (Sunspheres Powder von RÖHM & HAAS) | 2 | - |
| Poly-(C₁₀-C₃₀)-Alkylacrylate (Intelimer 13-1 von LANDEC) | 1 | 1 |
| Gemisch von Glycerylstearat, Behenalkohol, Natriumsulfat von Dicocoylethylendiamin PEG-15, Glyceryl Citrat Stearat | 2 | 2 |
| Isononylisononanoat | 4 | 4 |
| Cetylalkohol | 0,5 | 0,5 |
| Butylmethoxydibenzoylmethan | 3 | 3 |
| Ethylhexylsalicylat | 5 | 5 |
| Octocrylen | 7 | 7 |
| Duftstoff | 0,4 | 0,4 |
| Tocopherol | 0,2 | 0,2 |
| Copolymer-Acrylate | 0,7 | 0,7 |
| Triethanolamin | 0,2 | 0,2 |

2. Zusammensetzung nach Anspruch 1, **dadurch gekennzeichnet, dass** jede der Einheiten R₁-CO- und R₃-CO- einen Kokosfettsäurerest bezeichnen.

3. Zusammensetzung nach einem der vorangehenden Ansprüche, **dadurch gekennzeichnet, dass** für das Tensid-Paar der Formel (I), für jeden der Reste X und Y, die Summe von a und b einen Durchschnittswert von 10 bis 20 hat.

4. Zusammensetzung nach einem der vorangehenden Ansprüche, **dadurch gekennzeichnet, dass** für das Tensid-Paar der Formel (I) Y die Einheit SO₃M ist, wobei M ein Alkalimetallion ist, beispielsweise ein Natriumion.

5. Zusammensetzung nach einem der vorangehenden Ansprüche, **dadurch gekennzeichnet, dass** für das Tensid-Paar der Formel (I) n gleich 1 ist.

6. Zusammensetzung nach einem der vorangehenden Ansprüche, **dadurch gekennzeichnet, dass** das Tensid der Formel (I) die folgende Struktur besitzt:

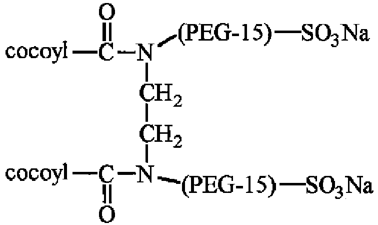

7. Zusammensetzung nach einem der vorangehenden Ansprüche, **dadurch gekennzeichnet, dass** das Tensid-Paar gemischt wird mit (a) einem C₆-C₂₂-Fettsäure- und Glycerylester, (b) einem C₆-C₂₂-Fettsäure- und Citronensäure- und Glyceryldiester, und (c) einem C₁₀-C₃₀-Fettalkohol.

8. Zusammensetzung nach einem der vorangehenden Ansprüche, **dadurch gekennzeichnet, dass** das Tensid-Paar in einem Gehalt von 0,01 bis 5 Gew.-%, bezogen auf das Gesamtgewicht der Zusammensetzung, vorzugsweise von 0,1 bis 3 Gew.-%, und bevorzugt von 0,2 bis 1,5 Gew.-% vorhanden ist.

9. Zusammensetzung nach einem der vorangehenden Ansprüche, wobei das Polymer ein Zahlenmittelmolekulargewicht von 3.000 bis 500.000 und besser von 4.000 bis 99.000 aufweist.

10. Zusammensetzung nach einem der vorangehenden Ansprüche, **dadurch gekennzeichnet, dass** das Polymer in der Fettphase zu mindestens 1 Gew.-% bei einer Temperatur über seiner Schmelztemperatur löslich ist.

11. Zusammensetzung nach einem der vorangehenden Ansprüche, **dadurch gekennzeichnet, dass** das Polymer eine Schmelztemperatur pF wie 30 °C ≤ pF < 50 °C aufweist.

12. Zusammensetzung nach einem der vorangehenden Ansprüche, enthaltend mindestens einen organischen Filter.

13. Kosmetisches Behandlungsverfahren einer Keratinsubstanz, wie Haut, Kopfhaut, Haare, Wimpern, Nägel, Schleimhäute, **dadurch gekennzeichnet, dass** auf die Keratinsubstanz eine Zusammensetzung nach einem der Ansprüche 1 bis 12 aufgebracht wird.

## Claims

1. A cosmetic or dermatological composition of oil-in-water type comprising, in a physiologically acceptable medium, at least one gemini surfactant of formula (I): 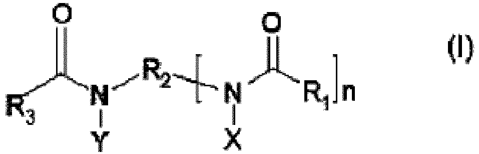 where:
- R₁ and R₃ each independently designate an alkyl radical having 1 to 25 carbon atoms;
- R₂ designates a spacer formed of a straight or branched alkylene chain having 1 to 12 carbon atoms;
- X and Y each independently designate a -(C₂H₄O)ₐ-(C₃H₆O)_{b}Z group where:
• Z designates a hydrogen atom or a radical-CH₂-COOM, -SO₃M, -P(O)(OM)₂, -C₂H₄-SO₃M, -C₃H₆-SO₃M or -CH₂(CHOH)₄CH₂OH where M is H or an alkaline or alkaline-earth or ammonium or alkanolammonium ion,
• a ranges from 0 to 15,
• b ranges from 0 to 10, and
• the sum a+b ranges from 1 to 25; and
- n ranges from 1 to 10,
in association with an efficient amount of at least one semi-crystalline polymer solid at ambient temperature and having a melting point lower than 70°C, comprising a) a polymeric backbone and b) at least one crystallisable organic side chain and/or a crystallisable organic sequence belonging to the backbone of said polymer, said polymer having a number average molecular weight of 2000 or higher,
said semi-crystalline polymer being selected from among homopolymers and copolymers resulting from the polymerisation of at least one monomer with crystallisable chain selected from among C₁₄-C₂₄ saturated alkyl (meth)acrylates;
said emulsion having a fatty phase content higher than 10 % by weight relative to the total weight of the composition and comprising more than 40 % by weight of non-volatile oil(s) relative to the weight of the fatty phase;
said composition differing from the following compositions A and B:
| **Ingredients** | **Composition** | **Composition** |
|---|---|---|
| | **A** (wt.%) | **B** (wt.%) |
| Disodium EDTA | 0.1 | 0.1 |
| Preserving agent | 1.25 | 1.25 |
| Glycerine | 4 | 4 |
| Propylene glycol | 4 | 4 |
| Water | 61.65 | 61.65 |
| 86 wt.% particle dispersion Styrene/acrylates copolymer (Sunspheres Powder by ROHM & HAAS) | 2 | - |
| Poly(C₁₀-C₃₀)alkylacrylates (Intelimer 13-1 by LANDEC) | 1 | 1 |
| Mixture of glyceryl stearate, behenic alcohol, sodium dicocoylethylendiamine PEG-15 sulfate, glyceryl stearate citrate | 2 | 2 |
| Isononyl isononaoate | 4 | 4 |
| Cetyl alcohol | 0.5 | 0.5 |
| Butyl methoxy dibenzoylmethane | 3 | 3 |
| Ethylhexyl salicylate | 5 | 5 |
| Octocrylene | 7 | 7 |
| Fragrance | 0.4 | 0.4 |
| Tocopherol | 0.2 | 0.2 |
| Copolymer acrylates | 0.7 | 0.7 |
| Triethanolamine | 0.2 | 0.2 |

2. The composition according to claim 1, **characterized by** the fact that each of the groups R₁-CO-and R₃-CO- designates a coconut fatty acids moiety.

3. The composition according to one of the preceding claims, **characterized by** the fact that for the gemini surfactant of formula (I), for each of the radicals X and Y, the sum of a and b has a mean value ranging from 10 to 20.

4. The composition according to any of the preceding claims, **characterized by** the fact that for the gemini surfactant of formula (I), Y is the group -SO₃M where M is an alkaline ion such as a sodium ion.

5. The composition according to any of the preceding claims, **characterized by** the fact that for the gemini surfactant of formula (I), n equals 1.

6. The composition according to any of the preceding claims, **characterized by** the fact that the surfactant of formula (I) has the following structure:

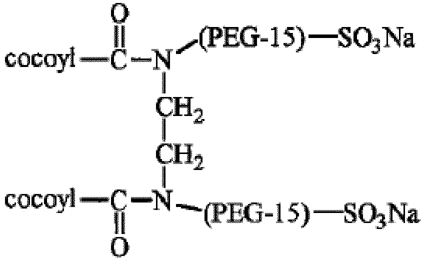

7. The composition according to any of the preceding claims, **characterized by** the fact that the gemini surfactant is mixed with (a) a C₆-C₂₂ fatty acid ester and glyceryl, (b)a diester of C₆-C₂₂ fatty acid and of citric acid and glycerol and (c) a C₁₀-C₃₀ fatty alcohol.

8. The composition according to any of the preceding claims, **characterized by** the fact that the gemini surfactant is contained in an amount ranging from 0.01 to 5 % by weight relative to the total weight of the composition, preferably ranging from 0.1 to 3 % by weight and more preferably ranging from 0.2 to 1.5 % by weight.

9. The composition according to any of the preceding claims, wherein the polymer has a number average molecular weight ranging from 3 000 to 500 000, and better from 4 000 to 99 000.

10. The composition according to any of the preceding claims, **characterized in that** the polymer is soluble in the fatty phase at least at 1 % by weight, at a temperature higher than its melting point.

11. The composition according to any of the preceding claims, **characterized in that** the polymer has a melting point m.p. such that 30°C ≤ m.p. < 50°C.

12. The composition according to any of the preceding claims containing at least one organic filter.

13. A method for the cosmetic treatment of keratinous material such as the skin, scalp, hair, eyelashes, eyebrows, nails or mucosa, **characterized by** the fact that a composition according to any of claims 1 to 12 is applied to said keratinous material.
